# EUROPÄISCHE PATENTSCHRIFT

(11) **EP 3 537 978 B1**
(45) Veröffentlichungstag und Bekanntmachung des Hinweises auf die Patenterteilung: **24.02.2021**
(21) Anmeldenummer: 17805103.3
(22) Anmeldetag: 13.11.2017
(51) Int. Cl.: A61B 6/03, A61B 6/00, H01J 35/06

(54) **COMPUTERTOMOGRAPH**
COMPUTER TOMOGRAPH
TOMOGRAPHE ASSISTÉ PAR ORDINATEUR

(30) Priorität: 12.11.2016 DE 102016013533
(43) Veröffentlichungstag der Anmeldung: 18.09.2019
(73) Patentinhaber: Esspen GmbH, 91054 Erlangen (DE)
(72) Erfinder: MOHAMMADI, Zahra, 91052 Erlangen (DE)
(74) Vertreter: Meyer, Rudolf
(86) Internationale Anmeldenummer: PCT/EP2017/001316
(87) Internationale Veröffentlichungsnummer: WO 2018/086744

(56) Entgegenhaltungen:
- WO-A1-2009/115982
- WO-A2-2007/038306
- DE-A1-102008 025 524
- US-A1- 2007 274 456
- US-A1- 2015 282 774
- US-A1- 2015 312 998

## Beschreibung

Die Erfindung betrifft ein Verfahren zum Betrieb eines Computertomographen, bei welchem eine Rotation eines Röntgenemitters synchron mit einem Röntgendetektor zur Röntgenbildgebung nicht erforderlich ist.

Kernkomponente üblicher Computertomographen ist die Gantry. Innerhalb des Gantry-Gehäuses rotieren mindestens eine Röntgenröhre und direkt gegenüberliegend die Detektoren zur Signalaufnahme. Mittels Hochspannung wird die bildgebende Röntgenstrahlung in der Röntgenröhre erzeugt. Der sogenannte Röntgengenerator enthält die gesamte Steuerung und Kontrolle. Die gewonnenen Messdaten, auch Rohdaten genannt, werden gesammelt an eine Recheneinheit übermittelt und von dieser direkt im Anschluss an die Aufnahme zu diagnostizierbaren Bildern rekonstruiert. Durch die rasante Entwicklung in der Informationstechnologie können leistungsstarke Computer diese Rechenleistung ausführen. In Mehrzeilen-Spiral-Computertomographen können Spezialkarten eingesetzt werden, welche über schnellere Signalprozessoren verfügen, um die Bildgenerierung in wenigen Sekunden durchzuführen.

Computertomographen mit einer rotierenden Röntgenquelle und zugehörigem Detektor sind beispielsweise in den Dokumenten DE 11 2014 003 207 T5, EP 1 617 764 B1, US 7 568 836 B2, WO 2006/015356 A2 und WO 2007/117677 A2 offenbart.

Aus der DE 10 2013 203 541 A1 ist ein Dual-Source-CT-System bekannt, bei welchem zwei Strahlenbündel derart durch Blenden begrenzt werden, dass die Strahlenbündel zumindest in einem Untersuchungsobjekt frei von gegenseitigen Schnittpunkten sind. Die Blenden können allgemein als Strahlungsbeeinflussungsmittel bezeichnet werden. Die Strahler-Detektor-Systeme einschließlich der Blenden stellen rotierende Komponenten des Dual-Source-CT-Systems dar.

Die EP 1 324 697 B1 offenbart einen CT-Scanner, welcher eine zeitkohärente großflächige Abdeckung bieten soll. In diesem Fall sind drei gemeinsam drehbare Röntgenstrahler-Detektor-Anordnungen vorhanden, wobei die verschiedenen Röntgenquellen in Richtung der Rotationsachse gegeneinander verschoben sind.

Die DE 28 52 968 A1 offenbart ein als "Schichtgerät zur Herstellung von Transversalschichtbildern eines Aufnahmeobjektes" bezeichnetes Tomographiegerät. Auch dieses Gerät weist drei zusammen rotierbare Strahler-Detektor-Anordnungen auf, welche in Richtung ihrer gemeinsamen Drehachse gegeneinander versetzt angeordnet sind.

Aus der EP 0 488 888 B1 ist eine weitere Tomographieanlage mit um eine Längsachse des Tomographen rotierenden Komponenten bekannt. Hierbei rotieren zwei Wagen, welche jeweils eine Röntgenstrahlquelle und ein zweidimensionales Sensorengitter tragen, simultan, so dass sich die Wagen konstant diametral gegenüberstehen.

Die US 2015/0305697 A1 offenbart ein Tomographiegerät, bei welchem die Strahler-Detektor-Anordnung starr, jedoch eine Filteranordnung drehbar ist. Die Strahler-Anordnung umfasst hierbei eine Vielzahl an Röntgenquellen, welche in Form eines um das zu untersuchende Volumen gelegten Ringes angeordnet sind. Hierbei kann eine Vielzahl von beispielsweise 1.000 Extraktionsgittern vorhanden sein, die jeweils einer Elektronenquelle zuzurechnen sind. Auf diese Wiese sollen Röntgenaufnahmen aus 1.000 verschiedenen Richtungen möglich sein, wobei die einzelnen Extraktionsgitter separat anzusteuern sind. Simultan mit der Ansteuerung der insgesamt ringförmig angeordneten Extraktionsgitter ist die Filteranordnung, das heißt eine Anordnung aus Strahlungsbeeinflussungsmitteln, um die Mittelachse des Tomographiegerätes zu drehen. Als Elektronen emittierende Materialien werden in der US 2015/0305697 A1 Kohlenstoff- oder Silizium-Nanoröhren vorgeschlagen.

Computertomographen mit einer rotierenden Gantry oder sonstigen rotierenden Komponenten, beispielsweise Filterkomponenten, weisen erhebliche Nachteile auf. Für eine gleichförmige und geometrisch präzise Rotation ist eine komplexe Mechanik mit einem hohen Platzbedarf erforderlich. Die mechanisch erfolgende Rotation bedingt weiter relativ langsame Rotationsgeschwindigkeiten und damit eine längere Aufnahmezeit, selbst bei mehreren Paaren von gegenüber angeordneten Röntgenquellen und Detektoren in einer Gantry. Solche Vorrichtungen sind sowohl in der Herstellung als auch durch die Störanfälligkeit der Mechanik im Unterhalt sehr kostenintensiv. Hervorzuheben ist insbesondere der hohe Energieverbrauch und enorme Platzbedarf, weswegen ein mobiler Einsatz, beispielsweise in Sanitätskraftwagen oder Feldlazaretten, solcher Computertomographen technisch allenfalls sehr aufwändig realisierbar ist.

Zur Behebung der genannten Nachteile wurde anstelle einer rotierenden Gantry für Computertomographen eine feste Reihenanordnung von Röntgenemittern vorgeschlagen. Bei einem solchen Computertomographen sind die Röntgenemitter auf den zu untersuchenden Körper ausgerichtet und werden jeweils einzeln elektrisch angesteuert. Die sequentielle Ansteuerung der Röntgenemitter ersetzt somit die bisher erforderliche Rotation einer Röntgenröhre. Eine Reihenanordnung von einzeln ansteuerbaren Röntgenemittern ist zum Beispiel auch in der DE 10 2011 076 912 B4 beschrieben.

Für Computertomographen mit einer festen Anordnung von Röntgenemittern sind insbesondere Röntgenemitter verwendbar, welche als Feldemissions-Röntgenröhren ausgebildet sind. Solche Röntgenemitter weisen beispielsweise Kathoden auf, welche Kohlenstoffnanoröhren enthalten. Ein stationärer Computertomograph dieser Bauart ist in US 7 751 528 B2 vorgeschlagen, der speziell für Röntgenaufnahmen der weiblichen Brust vorgesehen ist.

Mittlerweile ist die Röntgenbildgebung für die medizinische Diagnostik mittels Computertomographie etabliert. Beispielsweise ist bei Patienten mit Verdacht auf Schlaganfall oder allgemein bei Kopfverletzungen die Computertomographie meist das erste und deshalb wichtigste Mittel der Wahl. Auch in der Materialprüfung und beispielsweise zum Durchleuchten von verdächtigen Gegenständen hat sich die Computertomographie bereits bewährt.

Der Erfindung liegt die Aufgabe zugrunde, ein gegenüber dem Stand der Technik weiterentwickeltes Verfahren zum Betrieb eines Computertomographen anzugeben, welcher allgemein für die Röntgenbildgebung geeignet ist und auch als mobiles Gerät, beispielsweise in Sanitätskraftwagen oder Feldlazaretten, einsetzbar ist.

Diese Aufgabe wird erfindungsgemäß durch ein Verfahren zum Betrieb eines Computertomographen gemäß Anspruch 1 gelöst.

Ein zur Umsetzung des erfindungsgemäßen Verfahren geeigneter Computertomograph für die Röntgenbildgebung weist eine rotationsfeste Gantry auf. Hierbei stellt die Gantry eine Baugruppe dar, in welcher eine Mehrzahl von Röntgenemittern und eine Mehrzahl von Röntgendetektoren fest um eine geometrische Mittelachse, sich jeweils gegenüberliegend und in Richtung der Mittelachse gegeneinander versetzt angeordnet sind. Weiterhin sind Strahlungsbeeinflussungsmittel, nämlich Fokussierungselektroden, welche ebenfalls in fester Winkellage und damit fixierter Position relativ zu den Röntgenemittern und Röntgendetektoren, im Computertomographen angeordnet sind, der Gantry zuzurechnen. Hierbei wirken mehrere Elektronenemitter, das heißt Kathoden, welche zur Emission von Elektronen und dadurch, bei Auftreffen der Elektronen auf eine Anode, letztlich zur Erzeugung von Röntgenstrahlung vorgesehen sind, mit einem gemeinsamen Extraktionsgitter zusammen. Beispielsweise sind jeweils acht oder 24 Kathoden einem gemeinsamen Extraktionsgitter zugeordnet. Im Extremfall weist eine einzige Röntgenröhre mit einer Vielzahl an Kathoden lediglich ein einziges Extraktionsgitter auf.

Im Vergleich zu röntgentechnischen Geräten, bei welchen jede Kathode einem gesonderten Extraktionsgitter, welches separat anzusteuern ist, wie dies beispielsweise bei der US 2015/0305697 A1 der Fall ist, zugeordnet ist, ist bei dem nach dem erfindungsgemäßen Verfahren betreibbaren Computertomographen der apparative Aufwand drastisch reduziert. Dennoch kann der Computertomograph eine Vielzahl an Elektronenemittern und eine entsprechende Zahl, insbesondere mehr als 100, beispielsweise zwischen 200 und 400, an Röntgenemittern, aufweisen. Zudem entfällt die Notwendigkeit, beim Betrieb des Computertomographen Komponenten, etwa eine Strahler-Detektor-Anordnung und/oder eine Filteranordnung, in Rotation zu versetzen.

Die Röntgenemitter sind für eine gerichtete Emission und die zugehörigen Röntgendetektoren für eine Detektion von Röntgenstrahlen als Strahlenbündel vorgesehen. Diese Strahlenbündel weisen eine Richtung mit der maximalen Intensität der emittierten Röntgenstrahlung auf, wobei diese Richtung nachfolgend als Hauptemissionsrichtung bezeichnet wird. Eine solche Hauptemissionsrichtung ist bei allen Röntgenquellen gegeben, welche von einer Kugelstrahlquelle verschieden sind. In dem vorgeschlagenen Computertomographen ist die geometrische Form des Strahlenbündels durch die Konstruktionsweise der Röntgenquelle des betreffenden Röntgenemitters sowie durch Strahlungsbeeinflussungsmittel einstellbar. Der Begriff Strahlungsbeeinflussungsmittel kann sich allgemein auf Elektronenstrahlen und/oder Röntgenstrahlen beziehen. Neben Fokussierungselektroden, das heißt Mitteln zur Beeinflussung eines Elektronenstrahls, werden auch Blenden und Filter, welche auf die Röntgenstrahlung wirken, unter den Begriff Strahlungsbeeinflussungsmittel subsumiert. Beispielsweise ist in dem vorgeschlagenen Computertomographen ein Röntgenstrahlenbündel in Form eines Kegels oder eines Fächers gegeben. Beispielsweise ist in dem vorgeschlagenen Computertomographen eine Röntgenquelle in Form eines Brennflecks konstruktiv als Punktquelle oder Strichquelle oder als eine begrenzte Fläche auf einer Trägervorrichtung ausgebildet.

Die, bezogen auf die Mittelachse des Computertomographen, gegeneinander versetzte Anordnung der Röntgenemitter einerseits und Röntgendetektoren andererseits geht damit einher, dass die Hauptemissionsrichtung jeder Röntgenquelle die Mittelachse in einem Winkel verschieden von 90° schneidet.

Bei dem vorgeschlagenen Computertomographen wird zur Röntgenbildaufnahme jeweils ein Röntgenemitter zusammen mit mindestens einem gegenüber angeordneten Röntgendetektor sequentiell elektrisch angesteuert. Durch diesen Vorgang wird eine mechanische Rotation von Röntgenquellen und Detektoren ersetzt. Dabei liegt das Untersuchungsobjekt zwischen den Röntgenemittern und den Röntgendetektoren ein.

Beispielsweise kann eine Röntgenbildaufnahme dadurch erfolgen, dass nacheinander benachbarte Röntgenemitter zusammen mit einem gegenüber angeordneten Röntgendetektor sequentiell elektrisch angesteuert werden. Ebenso können Röntgenemitter und zugehörige Röntgendetektoren in beliebiger anderer Reihenfolge betrieben werden, wobei die Reihenfolge auch innerhalb der einzelnen Verschiebungsschritte variierbar ist. Die Auswahl eines bestimmten Bereiches eines röntgentechnisch zu untersuchenden Querschnittes (ROI = Region of Interest) kann dadurch erfolgen, dass nur diejenigen Röntgenemitter und Röntgendetektoren elektrisch angesteuert werden, welche auf die ROI ausgerichtet sind. Aus den so erhaltenen Röntgenbildern, welche Projektionsaufnahmen darstellen, lassen sich mittels computergestützter Verfahren, wie Tomosynthese oder gefilterter Rückprojektion (FBP = filtered back-projection), Querschnittsansichten und Volumenstrukturen des untersuchten Objektes erzeugen. Bei einer Projektion wird somit nur derjenige Einzelaufnahmebereich ausgewählt, welcher die für die computergestützte Bilderzeugung wesentlichen Informationen, das heißt Daten, enthält. Artefakte oder schlecht aufgelöste Bereiche werden damit vermieden. Dies verkürzt die für die computergestützte Bilderzeugung benötigte Zeit wesentlich. Somit kann die computergestützte Bilderzeugung auch mit Computern geringerer Rechnungsleistung schnell durchgeführt und zusätzlich eine noch bessere Bildauflösung erzielt werden, als wenn anstelle einer Mehrzahl von Röntgendetektoren ein einzelner Detektor verwandt wird.

Somit sind mit dem vorgeschlagenen Computertomographen bei gleichzeitig minimalem konstruktivem Aufwand hochaufgelöste Röntgenbildaufnahmen in einer bezüglich zum Stand der Technik verkürzten Aufnahmezeit möglich. Je mehr in der Gantry eine Mehrzahl von Röntgenemittern und Röntgendetektoren fest und sich jeweils gegenüberliegend angeordnet sind, desto höher ist die in der gesamten ROI erreichbare Bildauflösung.

Das Verfahren nach Anspruch 1 umfasst folgende Schritte:
- Ein erster Satz an Projektionsbildern, aufgenommen aus unterschiedlichen Projektionsrichtungen, wird durch den Computertomographen generiert,
- mindestens ein zusätzlicher Satz an Projektionsbildern wird aufgenommen, wobei die Projektionsrichtungen zumindest teilweise den Projektionsrichtungen des ersten Satzes an Projektionsbildern entsprechen,
- zwischen jeweils mindestens zwei aus übereinstimmender Projektionsrichtung aufgenommenen Projektionsbildern wird der Grad an Übereinstimmung festgestellt,
- weitere Projektionsbilder werden generiert, wobei die Häufigkeit der gewählten Projektionsrichtungen von dem Grad der Übereinstimmung der aus den betreffenden Projektionsrichtungen zu aufeinander folgenden Zeitpunkten aufgenommenen Projektionsbildern abhängt.

Das Untersuchungsobjekt kann sowohl den zu untersuchenden Körper, insbesondere ein Körperteil eines Patienten, als auch sonstige Gegenstände, beispielsweise Operationsbestecke, welche sich im Untersuchungsvolumen befinden, umfassen.

Durch die Abhängigkeit der Häufigkeit der bei Projektionsaufnahmen gewählten Winkeleinstellung, bezogen auf die Winkellage des innerhalb der Gantry angesteuerten Röntgenemitters, von dem Grad an Veränderungen, die aus der entsprechenden Winkellage heraus nacheinander aufgenommene Projektionsbilder zeigen, kann die Anzahl der Projektionsbilder, welche für die Erzeugung aussagekräftiger, auch sich zeitlich rasch ändernder Schnittbilder erforderlich ist, minimiert werden. Insbesondere werden umso häufiger Projektionsaufnahmen aus einer bestimmten Projektionsrichtung erstellt, je geringer der Grad an Übereinstimmung zwischen zu aufeinander folgenden Zeitpunkten aus der entsprechenden Projektionsrichtung aufgenommenen Projektionsbildern ist. Die Winkelrelationen zwischen den Röntgenemittern, welche nacheinander angesteuert werden, sind hierbei nicht fest vorgegeben, sondern ergeben sich erst durch die Auswertung der Projektionsbilder während des Betriebs des Computertomographen.

Allgemein ist die Anzahl aller Röntgenemitter in dem vorgeschlagenen Computertomographen mindestens gleich der Anzahl von Projektionen für eine solche computergestützte Bilderzeugung. Sind einem Röntgenemitter mehrere Röntgendetektoren zugeordnet, kann mit einem Röntgenemitter mehr als eine Projektionsaufnahme erzeugt werden.

Neben der Zuordnung eines einzelnen Röntgenemitters nur zu einer bestimmten Anzahl von Röntgendetektoren beruht die Erfindung auf dem Grundgedanken der jeweils in Richtung der Mittelachse gegeneinander versetzten Anordnung der betreffenden Röntgenemitter und Röntgendetektoren zueinander.

Mit einer solchen Anordnung sind tote Winkel um die Axialrichtung vermeidbar. Sind beispielsweise sowohl die Röntgenemitter als auch die Röntgendetektoren jeweils in einem Bogen von mehr als 180° um die Axialrichtung angeordnet, so ist dies nur bei einem axialen Versatz der Röntgenemitter und der Röntgendetektoren zueinander möglich. Nur mit dem vorgeschlagenen Computertomographen, welcher dieses wesentliche konstruktive Merkmal aufweist, können die Röntgenemitter und die Röntgendetektoren auch vollständig umschließend um die Axialrichtung und damit um den vorgesehenen Untersuchungsbereich angeordnet werden. Damit sind besonders vorteilhaft bei dem vorgeschlagenen Computertomographen mit diesem konstruktiven Merkmal sowohl die Anordnungsgeometrien der Röntgenemitter als auch der Röntgendetektoren um die Axialrichtung frei wählbar.

Beispielsweise sind in einer Klasse von Ausführungsformen des vorgeschlagenen Computertomographen die Röntgenemitter kreisförmig und die Röntgendetektoren in Winkelanordnungen um die Axialrichtung angeordnet. Ist der vorgeschlagene Computertomograph beispielsweise zur Untersuchung der menschlichen Brust oder zur Materialprüfung von Werkstücken vorgesehen, ist es nicht erforderlich, dass die Röntgenemitter oder Röntgendetektoren die Mittelachse vollständig umschließen.

Beispielsweise ist bei dem vorgeschlagenen Computertomographen die Gantry auf einer Gerätebasis montiert. Zweckmäßigerweise sind in der Gerätebasis Vorrichtungen zur Stromversorgung und zur elektronischen Steuerung sowie der Computer verbaut. Der vorgeschlagene Computertomograph ist in einer bevorzugten Bauform als transportables Gerät besonders einfach realisierbar.

Bei der Wahl eines Röntgenstrahlenbündels in Form eines Kegels oder eines Fächers mit einer zur Mittelachse parallelen Fächerebene und bei einer hinreichenden Breite der Röntgenstrahlungsabsorptionsfläche der Röntgendetektoren ist mit dem vorgeschlagenen Computertomographen mit einer ortsfesten Gantry die Abbildung einer ROI bei einem unbewegten Objekt realisierbar.

Nachfolgend wird auf einzelne vorteilhafte Weiterbildungen des vorgeschlagenen Computertomographen eingegangen.

In einer Ausführung ist der vorgeschlagene Computertomograph durch eine Gantry weitergebildet, welche in Richtung der Mittelachse verschiebbar ist. In dieser Ausführung des vorgeschlagenen Computertomographen führen bei einer Röntgenbildaufnahme die Gantry und das Untersuchungsobjekt eine Relativbewegung in Richtung der Mittelachse zueinander aus. Dazu ist die Gantry beispielsweise auf einer Anordnung von geraden Führungsschienen auf einer Gerätebasis montiert, wobei die geraden Führungsschienen parallel zu der Mittelachse sind.

Mit dem vorgeschlagenen Computertomographen in dieser Ausführung ist bei einer Röntgenbildaufnahme eine Abdeckungsbreite von mindestens 30 cm in Richtung der Mittelachse durch Verschieben der Gantry erreichbar. In dieser Weiterbildungsform ist der vorgeschlagene Computertomograph damit besonders für die computertomographische Röntgenbildgebung des menschlichen Kopfes oder der Brust geeignet.

Zur computertomographischen Röntgenbilderzeugung kann die Gantry schrittweise, in alternativer Verfahrensführung kontinuierlich, in Richtung der Mittelachse über das Untersuchungsobjekt geführt werden. Bei jedem Schritt erfolgt dann wie zuvor beschrieben eine Röntgenbildaufnahme durch sequentielle elektrische Ansteuerung der einzelnen Röntgenemitter zusammen mit mindestens einem gegenüber angeordneten Röntgendetektor. Alle Einzelschritte decken hierbei die ROI in Richtung der Mittelachse vollständig ab. Aus den so erhaltenen Röntgenbildern, welche Projektionsaufnahmen darstellen, lassen sich mittels computergestützter Bilderzeugung Querschnittsansichten und Volumenstrukturen des untersuchten Objektes erzeugen. Somit ist bei dem vorgeschlagenen Computertomographen in dieser Ausführung die ROI nicht nur durch eine selektive Ansteuerung der Röntgenemitter und Röntgendetektoren auswählbar, sondern auch durch das jeweils gewählte Verschiebungsintervall der Gantry in Richtung der Mittelachse.

Durch Wahl eines Röntgenstrahlenfächers in Form eines Fächers mit einer Fächerebene und einer Hauptemissionsrichtung senkrecht zur Röntgenstrahlungsabsorptionsfläche des Röntgendetektors lässt sich bei dieser Ausführung des vorgeschlagenen Computertomographen besonders vorteilhaft die Röntgenstrahlexposition des Untersuchungsobjektes auf den gewünschten geometrischen Teilausschnitt der ROI beschränken. Weiter ist so die Wahl von Röntgendetektoren mit einer geringeren Breite der Röntgenstrahlungsabsorptionsfläche möglich, wobei auch der Fertigungsaufwand erheblich gesenkt ist. Mit steigender Anzahl der geometrischen Teilausschnitte pro Länge der ROI in Richtung der Mittelachse steigt auch die Auflösung der durch die computergestützte Bilderzeugung erzeugten Querschnittsansichten und Volumenstrukturen des untersuchten Objektes. Auch in dieser Bauform ist der vorgeschlagene Computertomograph als Ganzes vorzugsweise als mobiles Gerät ausgebildet.

In einer bevorzugten Weiterbildung umschließen die Röntgenemitter und die Röntgendetektoren des vorgeschlagenen Computertomographen die Mittelachse vollständig. Dies hat den Vorteil, dass eine Röntgenbildgebung eines beliebigen Ausschnitts (ROI) des untersuchten Objektes in gleichbleibender, hoher Qualität möglich ist.

Bilden hierbei die Röntgenemitter und die Röntgendetektoren einen Kreis um die Mittelachse aus, so wird damit zusätzlich eine besonders gleichförmige Auflösung des Röntgenbildes erzielt. In dieser Ausführungsform ist der vorgeschlagene Computertomograph insbesondere für die Röntgenbildgebung des menschlichen Kopfes geeignet. Besonders vorteilhaft ist somit auch bei einer nur teilweisen Ansteuerung von Röntgenemittern eine ROI in allen Richtungen um die Axialrichtung, das heißt Längsachse des Computertomographen, mit einer gleichbleibenden, hohen Qualität und hohen Auflösung frei auswählbar.

Für eine hohe Auflösung des Röntgenbildes ist eine kreisförmige Anordnung der Einzelröntgenemitter und der Einzeldetektoren nicht zwingend erforderlich. In einer weiteren Ausführungsform des vorgeschlagenen Computertomographen sind die Einzelröntgenemitter oder Detektoren in mindestens drei gleichartigen, insbesondere gleich langen, Zeilen um die Mittelachse angeordnet, wobei die Zeilen ein regelmäßiges Polygon ausbilden. Die Auflösung des Röntgenbildes wird verbessert, desto mehr Seiten das regelmäßige Polygon, zu welchem die Multi-Röntgenemitter-Anordnung oder die Multi-Detektor-Anordnung ausgebildet ist, aufweist. Mit Anzahl der Zeilen steigt jedoch der Fertigungsaufwand. Als optimal hinsichtlich der Auflösung des Röntgenbildes und des Fertigungsaufwandes hat sich für das Polygon ein Hexagon oder Oktagon oder Decagon erwiesen.

Nachfolgend wird auf die vorteilhaften Ausbildungen der Röntgenemitter und der Röntgendetektoren des vorgeschlagenen Computertomographen eingegangen. Vorzugsweise weisen die Röntgenemitter in der Gantry Kathoden zur Feldemission von Elektronen auf, wobei die Kathoden Kohlenstoffnanoröhren enthalten. Die Kohlenstoffnanoröhren dienen als Kaltkathoden, um Elektronen zu erzeugen, die dann beschleunigt werden, um die Anode als eigentliche Röntgenquelle des Röntgenemitters zu beschießen. In dieser Weiterbildung des vorgeschlagenen Computertomographen sind die Röntgenemitter als Feldemissions-Röntgenstrahler ausgebildet. Durch das elektronische Ein- und Ausschalten einer einzelnen Kathode wird ein bildgebender Röntgenstrahl auf der Anode erzeugt. Solche Röntgenemitter lassen sich besonders klein gestalten und auf einem gemeinsamen Träger aufbringen, welcher von einer einzigen Vakuumröhre umschlossen ist; eine solche Anordnung stellt eine MBFEX-Röhre (MBFEX = Multibeam Field Emission X-Ray) dar, bei welcher wiederum eine kompaktere Bauform erzielbar ist. Kohlenstoffnanoröhren weisen einen niedrigen Feldstärke-Schwellenwert für von weniger als 2 V µm⁻¹ für die Feldemission von Elektronen auf. Daher ist der Betrieb des vorgeschlagenen Computertomographen mit einer Stromversorgung möglich, welche nur eine verhältnismäßig geringe Leistungsstärke aufweist.

Kohlenstoffnanoröhren werden verallgemeinert als Nanostäbchen bezeichnet. An Stelle von Kohlenstoffnanoröhren oder zusätzlich zu Kohlenstoffnanoröhren können Elektronenemitter des Computertomographen auch andere Nanostäbchen aufweisen. Die Nanostäbchen können hierbei eine in sich einheitliche oder eine uneinheitliche Zusammensetzung aufweisen und entweder als Hohlkörper, das heißt Röhren, oder massiv ausgebildet sein.

Beispielsweise können die Nanostäbchen, insbesondere Nanoröhren, aus Metalloxiden gebildet sein. Grundsätzlich sind Nanostäbchen aus Metalloxiden - ebenso wie Nanodrähte, welche im vorliegenden Fall nicht relevant sind - zum Beispiel aus der Veröffentlichung "Theme issue: inorganic nanotubes and nanowires", Journal of Materials Chemistry, 2009, 19, 826 - 827 bekannt. In dieser Veröffentlichung sind unter anderem TiO₂, ZnO und Al₂O₃ und als Materialien, aus welchen Nanoröhren gebildet sein können, genannt.

Zur Herstellung eines im zur Durchführung des erfindungsgemäßen Verfahrens vorgesehenen Computertomographen zum Einsatz kommenden Elektronenemitters sind Metalloxide, beispielsweise Titanoxid, Zinkoxid oder Manganoxid sowohl in reiner als auch in dotierter Form geeignet. Ebenso können sonstige Materialien, aus welchen die Nanostäbchen aufgebaut sind oder welche in den Nanostäbchen enthalten sind, beispielsweise Metalle, Sulfide, Nitride oder Carbide, entweder in purer oder in dotierter Form vorliegen.

Sofern der Elektronenemitter ein Sulfid enthält, kann es sich beispielsweise um ein Metallsulfid, insbesondere Molybdändisulfid, handeln. Als Nitride, aus welchen Nanostäbchen des Elektronenemitters vollständig oder teilweise aufgebaut sein können, sind insbesondere Bornitrid, Aluminiumnitrid, Kohlenstoffnitrid und Galliumnitrid zu nennen. Als Carbid ist insbesondere Siliziumcarbid zur Herstellung der Nanostäbchen, insbesondere Nanoröhren, geeignet. Ebenso sind Nanostäbchen, insbesondere in der Form von Nanoröhren, aus Silizium, optional mit Dotierungselementen, herstellbar.

Auch die Verwendung von Nanostäbchen, welche Cer oder Lanthan enthalten, ist im Rahmen der Herstellung des Elektronenemitters möglich. In diesem Zusammenhang wird beispielhaft auf die Patentanmeldung WO 2014/076693 A1 hingewiesen.

Als Ausgangsprodukte zur Herstellung der Nanostäbchen, welche im Betrieb des Elektronenemitters Elektronen emittieren, sind auch stabförmige, optional hohle, Elemente aus polymeren Materialien geeignet. Die Nanostäbchen des Elektronenemitters können aus Ausgangsprodukten, welche komplett aus polymeren Materialien aufgebaut sind, oder aus Ausgangsprodukten, welche lediglich partiell, insbesondere in Form einer Beschichtung, Polymermaterialien aufweisen, gefertigt sein.

Innerhalb des Computertomographen können Elektronenemitter einheitlicher oder uneinheitlicher Gestaltung vorhanden sein. Ebenso können die zugehörigen Fokussierungselektroden entweder im kompletten Computertomographen einheitlich oder uneinheitlich gestaltet sein. Somit existieren vier Möglichkeiten, Elektronenemitter mit Fokussierungselektroden zu kombinieren:
- Die Kombination einheitlicher Emitter mit einheitlichen Fokussierungselektroden,
- die Kombination einheitlicher Emitter mit unterschiedlichen Typen von Fokussierungselektroden,
- die Kombination Emitter unterschiedlicher Art mit einheitlichen Fokussierungselektroden,
- die Kombination unterschiedlicher Emitter mit unterschiedlichen Typen von Fokussierungselektroden.

Verschiedene Emitter können sich hierbei hinsichtlich ihrer Geometrie und/oder hinsichtlich ihrer Materialien voneinander unterscheiden. Die Emitter, insbesondere in Form von Kohlenstoffnanoröhren enthaltenden Emittern, sind in hoher, einheitlicher Qualität herstellbar. Details zur möglichen Herstellung der Emitter sind in der beim DPMA eingereichten Patentanmeldung Nr. 10 2016 013 279.5 sowie in der deren Priorität beanspruchenden, beim EPA am 08. November 2017 eingereichten PCT-Anmeldung offenbart. Derartige Emitter zeichnen sich insbesondere durch äußerst geringe Alterungseffekte aus.

Auch die Verwendung von Dispenserkathoden, wie sie grundsätzlich zum Beispiel aus der DE 10 2011 076 912 B4 bekannt sind, ist möglich.

Von den Elektronenemittern ausgehende, das Extraktionsgitter passierende, durch die Fokussierungselektroden beeinflussten Elektronenstrahlbündel treffen auf eine Anode, welche in bevorzugter Ausgestaltung als starre, flüssigkeitsgekühlte Anode ausgebildet ist. Hierbei fließt ein Kühlmittel, insbesondere in Form eines leitfähigen Öls, vorzugsweise konzentrisch zur Mittelachse der langgestreckten, stabförmigen oder gebogenen Anode durch einen Kanal mit ringförmigem Querschnitt, welcher innerhalb der Anode gebildet ist. Mittig innerhalb dieses Kanals befindet sich in dieser Ausgestaltung ein weiterer Kanal, durch den das Kühlmittel zurück geleitet wird. Die Anode hat somit die Gestalt und Funktion einer Kühlfingers, an dessen Ende das eingeleitete Kühlmittel umgeleitet wird.

Die Anode ist beim Betrieb des Computertomographen auf Hochspannung in der Größenordnung von 100 kV gelegt. Zusätzlich zur Variation des Emissionsstroms der Kathoden ist auch eine Variation der Anodenspannung möglich, wobei beide Größen - Emissionsstrom und Anodenspannung - sehr schnell veränderbar sind. Durch Multiplikation der beiden veränderbaren Parameter Emissionsstrom und Anodenspannung ist eine Vielzahl unterschiedlicher Einstellungen von Betriebsparametern des Computertomographen während ein und derselben Untersuchung möglich. Dies betrifft sowohl die Frequenz der emittierten Röntgenstrahlung als auch die pro Puls emittierte Röntgendosis. Statt einer Dual-Energy-CT-Anlage, wie prinzipiell aus dem eingangs zitierten Stand der Technik bekannt, stellt der Computertomograph damit ein Multi-Energy-Computertomographiegerät dar. Beispielsweise wird die emittierte Röntgenstrahlung, was die Parameter Wellenlänge und Dosis pro Puls betrifft, in mindestens 100 Stufen während der Untersuchung eines Untersuchungsobjektes variiert. Von besonderem Vorteil ist zugleich die Tatsache, dass die Röntgendetektoren samt zugehöriger Auswertetechnik im Vergleich zu herkömmlichen Dual-Energy-Anlagen, welche mittels der Detektoren Messungen durchführen, die sich auf zwei verschiedene Wellenlängen beziehen, besonders einfach gestaltet sein können.

Die Röntgendetektoren in der Gantry sind beispielsweise als Photonenzähldetektoren oder als einzelne Flachbilddetektoren für Röntgenstrahlen oder als einzelne Photodioden, wie beispielsweise direkte Halbleiterdetektoren (SSD = solid state detector), ausgebildet. Dies gestattet eine besonders einfache elektronische Ansteuerung der Röntgendetektoren zusammen mit den jeweils zugeordneten Röntgenemittern.

Vorzugsweise weisen die Röntgendetektoren direkte Halbleiterdetektoren zur Detektion von Röntgenstrahlung auf. Sind hierbei die Röntgendetektoren beispielsweise als Flachbilddetektoren ausgebildet, wandeln diese Röntgenstrahlungssignale in elektrische Signale für jedes Pixel auf der Röntgenstrahlungsabsorptionsfläche um. Aus diesen elektrischen Signalen ist dann ein Röntgenbild generierbar. Mit solchen Röntgendetektoren ist eine sehr hohe Auflösung des Röntgenbildes realisierbar. Solche Röntgendetektoren sind auch besonders einfach in einer gemeinsamen Anordnung integrierbar, wobei diese Anordnung eine Detektor-Baugruppe darstellt.

In einer besonders bevorzugten Bauform des vorgeschlagenen Computertomographen sind jeweils die Röntgenemitter in einer MBFEX-Röhre und die Röntgendetektoren in einer Detektor-Baugruppe in der Gantry fest angeordnet. Die Anordnung der MBFEX-Röhre und der Detektor-Baugruppe zueinander ist so gestaltet, dass die Hauptemissionsrichtung jeder Anode eines jeden Röntgenemitters die Mittelachse in einem Winkel verschieden von 90° schneidet. Damit sind jeder Röntgenemitter und jeder Röntgendetektor in Richtung der Mittelachse des Computertomographen gegeneinander versetzt angeordnet. In dieser Weiterbildung kann der vorgeschlagene Computertomograph besonders kompakt und stabil realisiert werden.

Durch die Verwendung von Röntgendetektoren mit hoher Framerate und voller Auflösung ist es möglich, in weniger als 20 Sekunden einen kompletten Satz hochauflösender Projektionsbilder zu erstellen, ähnlich wie bei der standardmäßigen CT. Mit dem vorgeschlagenen Computertomographen ist damit in wenigen Sekunden eine hochaufgelöste vollständige Röntgenbildaufnahme realisierbar, was insbesondere bei der Untersuchung von unruhigen Patienten von besonderem Vorteil ist.

Im Gegensatz zu bekannten Computertomographen mit einer rotierenden Anordnung von Röntgenquelle und einem Detektor ist bei Röntgenbildaufnahmen mit dem vorgeschlagenen Computertomographen eine Brennfleckvergrößerung um die Axialrichtung aufgrund bewegter Komponenten prinzipbedingt ausgeschlossen.

Der vorgeschlagene Computertomograph, insbesondere in seinen Weiterbildungen, zeichnet sich durch eine sehr kompakte und robuste Bauweise aus. Besonders vorteilhaft erfordert eine Röntgenbildaufnahme keine Bewegung des Patienten durch die Gantry.

Der vorgeschlagene Computertomograph, insbesondere mit Röntgenemittern, welche Kaltkathoden mit Kohlenstoffnanoröhren aufweisen, weist im Vergleich zu den aktuell auf dem Markt erhältlichen Computertomographen folgende Vorteile auf:
- Reduktion der Strahlungsdosis für die Patienten,
- Steigerung der Sensitivität und Spezifität von bildgebenden Geräten,
- höhere Leistung,
- weniger Gewicht und Stellfläche,
- eine verbesserte Mobilität für Chirurgen/Ärzte,
- Komplettbetreuung bedürftiger Patienten,
- Verbesserung der Qualität bzw. Senkung der Kosten (speziell die Anschaffungs- und Betriebskosten für solche medizinischen Bildgebungssysteme) von Gesundheitsfürsorge-Dienstleister.

Die Verwendung des vorgeschlagen Computertomographen ist keineswegs auf die medizinische Diagnostik beschränkt. Der vorgeschlagene Computertomograph ist beispielsweise auch für die Röntgenbildgebung von nichtbelebten Objekten, beispielsweise zur Werkstückprüfung oder Produktprüfung oder zur Inhaltsprüfung von verschlossenen Behältern, geeignet.

Nachfolgend wird die Erfindung anhand einer Zeichnung näher erläutert, in welcher mehrere Ausführungsbeispiele von Computertomographen zusammengefasst sind. Hierin zeigt:
- Fig. 1: einen Computertomograph **1** im Querschnitt senkrecht zur Mittelachse **z** seiner Gantry **2,**
- Fig. 2: einen Computertomograph **1** im Querschnitt parallel zur Mittelachse **z** seiner Gantry **2,**
- Fig. 3: schematisch verschiedene Ausführungen eines Computertomograph **1** bezüglich der Anordnungen von Röntgenemittern **3** und Röntgendetektoren **4,**
- Fig. 4: zwei Ausführungen eines Computertomograph **1** mit die Mittelachse **z** vollständig umschließenden Röntgenemittern **3** und Röntgendetektoren **4.**
- Fig. 5: einen Computertomograph **1** mit schematischer Darstellung eines elektronischen Mehrkanal-Steuersystems **12,**
- Fig. 6: Merkmale einer MBFEX-Röhre **9** eines Computertomographen **1** samt zugehöriger Ansteuerung in schematischer Darstellung,
- Fig. 7: in einer Ansicht entsprechend Fig. 3 einen Röntgenemitter **3** eines Computertomographen **1** sowie ein zu untersuchendes Objekt,
- Fig. 8: das mit der Anordnung nach Fig. 7 zu untersuchende Objekt in zwei verschiedenen Zuständen,
- Fig. 9: in einem Diagramm die Veränderung der Parameter Emissionsstrom und Anodenspannung des Computertomographen **1** nach Fig. 6,
- Fig. 10: in einem Diagramm die Abhängigkeit eines mit dem Computertomographen **1** nach Fig. 6 aufgenommenen Detektorsignals von physikalischen Eigenschaften eines Untersuchungsobjektes bei drei verschiedenen Einstellungen des Röntgenemitters **3.**

Alle nachfolgend anhand einer Zeichnung erläuterten Ausführungsbeispiele des vorgeschlagenen Computertomographen **1** weisen eine rotationsfeste Gantry **2** auf. In der Gantry **2** sind eine Mehrzahl von Röntgenemittern **3** und Röntgendetektoren **4** fest um eine geometrische Mittelachse **z,** das heißt nicht drehbar, sich jeweils gegenüberliegend und in Richtung der Mittelachse z gegeneinander versetzt angeordnet. Zur Röntgenbildaufnahme wird bei allen Ausführungsbeispielen jeweils ein Röntgenemitter **3** zusammen mit einem gegenüber angeordneten Röntgendetektor **4** sequentiell elektrisch angesteuert.

Die Röntgenemitter **3** weisen in allen Ausführungsbeispielen Kathoden **5** zur Feldemission von Elektronen auf, um Elektronen zu erzeugen, die dann beschleunigt werden, um die Anode **6** als eigentliche Röntgenquelle des jeweiligen Röntgenemitters **3** zu beschießen. Die Kathoden **5** der Röntgenemitter **3** enthalten Kohlenstoffnanoröhren. Somit sind die Röntgenemitter **3** als einzelne Feldemissions-Röntgenstrahler ausgebildet. Die Röntgenemitter **3** sind auf einem gemeinsamen Träger fest aufgebracht und in einer Vakuumröhre **7** verbaut. In der Vakuumröhre **7** sind Röntgenfenster **8** eingelassen, durch welche die erzeugte Röntgenstrahlung austreten kann. Damit entspricht diese Anordnung der Röntgenemitter **3** einer MBFEX-Röhre **9.**

Die Röntgendetektoren **4** sind in allen Ausführungsbeispielen als Flachbilddetektoren ausgebildet, welche direkte Halbleiterdetektoren zur Detektion von Röntgenstrahlung aufweisen. Die Röntgendetektoren **4** sind in einer Detektor-Baugruppe **10** fest angeordnet.

In allen Ausführungsbeispielen des vorgeschlagenen Computertomographen **1** sind die jeweilige MBFEX-Röhre **9** und die Detektor-Baugruppe **10** in einer solchen Weise zueinander fest in der Gantry **2** angeordnet, dass die Hauptemissionsrichtung **e** jeder Anode **6** eines jeden Röntgenemitters **3** die Mittelachse z in einem Winkel verschieden von 90° schneidet.

Alle Ausführungen des vorgeschlagenen Computertomographen **1** sind als transportable Geräte für computertomographische Röntgenbildgebung von Körperteilen eines Patienten, insbesondere des Kopfes und der Brust, vorgesehen. Bei einer Röntgenbildaufnahme einer menschlichen Extremität oder eines menschlichen Kopfes oder einer menschlichen Brust befindet sich diese zwischen den Röntgenemittern **3** und Röntgendetektoren **4,** vorzugsweise im Bereich um die Mittelachse **z.**

In allen Ausführungsbeispielen ist die Gantry **2** auf einer Gerätebasis **11** montiert. In der Gerätebasis **11** ist ein elektronisches Mehrkanal-Steuersystem **12** verbaut, wobei das elektronische Mehrkanal-Steuersystem **12** zur Steuerung des Computertomographen **1,** der Röntgenemitter **3** und der Röntgendetektoren **4,** Erfassung der Daten für die Röntgenbildgebung und der computergestützten Bilderzeugung daraus, für die Datenspeicherung und für die Datenausgabe und Röntgenbildausgabe vorgesehen ist.

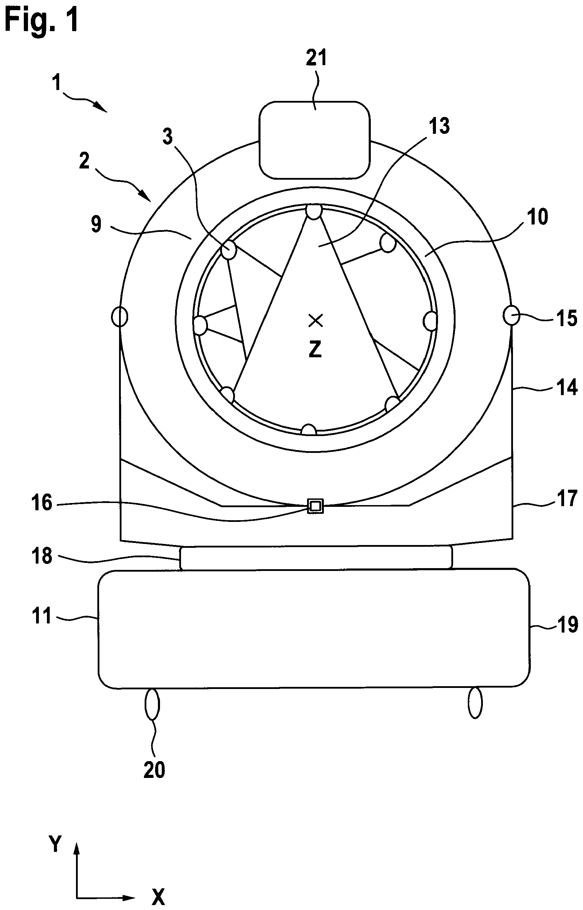

Die Fig. 1 zeigt ein erstes Ausführungsbeispiel des Computertomographen **1** im Querschnitt senkrecht zur Mittelachse **z** seiner Gantry **2** mit Sicht auf die Röntgenemitter **3** in der MBFEX-Röhre **9.** Die Röntgenemitter **3** sind kreisförmig vollständig umschließend um die Mittelachse **z** angeordnet, dementsprechend ist die MBFEX-Röhre **9** auch kreisförmig ausgebildet. Die Kathoden **5** und die Anoden **6** der Röntgenemitter **3** sowie die Vakuumröhre **7** sind in der Fig. 1 nicht sichtbar. Die Röntgendetektoren **4** sind in der Fig. 1 ebenfalls nicht sichtbar und in der Detektor-Baugruppe **10** verbaut, wobei in diesem Ausführungsbeispiel die Detektor-Baugruppe **10** ebenfalls kreisförmig vollständig umschließend um die Mittelachse **z** gestaltet ist. Die geometrische Mittelachse **z** verläuft in diesem Ausführungsbeispiel durch beide Kreismittelpunkte der MBFEX-Röhre **9** und Detektor-Baugruppe **10** beziehungsweise der jeweiligen Anordnungen aus Röntgenemittern **3** und Röntgendetektoren **4,** so dass in diesem Ausführungsbeispiel beide Kreismittelpunkte mit ihrer Position den Verlauf der Mittelachse **z** definieren.

In diesem Ausführungsbeispiel sind die Anoden **6** eines jeden Röntgenemitters **3** in der Weise konstruiert, dass diese jeweils einen Röntgenstrahlenfächer **13** mit einer Fächerebene und der Hauptemissionsrichtung **e** senkrecht zur Röntgenstrahlungsabsorptionsfläche des jeweiligen Röntgendetektors **4** erzeugen.

Somit ist in diesem Ausführungsbeispiel jede ROI im Innenbereich der Gantry **2** um die Mittelachse **z** vollständig, gleichförmig und mit hoher Auflösung bei gleichzeitiger verhältnismäßig geringer Röntgenstrahlexposition des Untersuchungsobjektes abbildbar, wie dies an den für einzelne Röntgenemitter **3** eingezeichnete Röntgenstrahlenfächer **13** der emittierten Röntgenstrahlung zeichnerisch verdeutlicht ist. Der vorgeschlagene Computertomograph in diesem Ausführungsbeispiel, insbesondere die Gantry **2**, zeichnet sich durch eine besonders kompakte Bauform aus.

Die Gerätebasis **11** des Computertomographen **1** in diesem Ausführungsbeispiel weist eine Halterung **14** mit zwei Schwenkpunkten **15** und mit einer Arretierungsvorrichtung **16,** eine Verschiebevorrichtung **17,** eine Hebevorrichtung **18** und ein Gehäuse **19** auf feststellbaren Rollen **20** auf. Die Gantry **2** ist in den Schwenkpunkten **15** der Halterung **14** schwenkbar auf der Gerätebasis **11** montiert und über die Arretierungsvorrichtung **16** arretierbar. Die Halterung **14** ist auf der Verschiebevorrichtung **17** aufgebracht, wobei die Verschiebevorrichtung **17** zur Verschiebung der Gantry **2** in Richtung der Mittelachse **z** vorgesehen ist. Die Hebevorrichtung **18,** welche auf dem Gehäuse **19** angebracht ist, ist im Falle der Röntgenbildgebung für den menschlichen Kopf zur Höheneinstellung der Gantry **2** vor Beginn der computertomographischen Röntgenbildaufnahme vorgesehen. In dem Gehäuse **19** ist das elektronisches Mehrkanal-Steuersystems **12** verbaut. An der Gantry **2** ist ein Bildschirm **21** montiert, welcher zur Bedienung des Mehrkanal-Steuersystems **12** und der Wiedergabe des computertomographisch erzeugten Röntgenbildes vorgesehen ist. Der Computertomograph **1** ist in diesem Ausführungsbeispiel als mobiles Gerät gestaltet und auf den feststellbaren Rollen **20** zu jedem gewünschten Untersuchungsstandort verschiebbar.

Die Verschiebevorrichtung **17** weist eine Anordnung von geraden Führungsschienen und einen Elektromotor auf. Die geraden Führungsschienen sind parallel zu der Mittelachse **z.** Auf den Führungsschienen ist die Gantry **2** mittels des Elektromotors in Richtung der Mittelachse **z** verschiebbar. In der Fig. 1. sind die Führungsschienen und der Elektromotor nicht sichtbar. In dieser Ausführung des vorgeschlagenen Computertomographen **1** führen bei einer Röntgenbildaufnahme, beispielsweise des menschlichen Kopfes, die Gantry **2** und das Untersuchungsobjekt eine Relativbewegung in Richtung der Mittelachse **z** zueinander aus. Zur computertomographischen Röntgenbilderzeugung wird die Gantry **2** schrittweise in Richtung der Mittelachse **z** über das Untersuchungsobjekt geführt. Bei jedem Schritt erfolgt dann eine Röntgenbildaufnahme durch sequentielle elektrische Ansteuerung der einzelnen Röntgenemitter **3** zusammen mit mindestens einem gegenüber angeordneten Röntgendetektor **4.** Alle Einzelschritte decken hierbei die ROI in Richtung der Mittelachse **z** vollständig ab. Bei diesem Ausführungsbeispiel ist somit bei Röntgenbildaufnahmen eine Abdeckungsbreite von 30 cm in Richtung der Mittelachse **z** erreichbar. Bei einer solchen Röntgenbildaufnahme mit einer schrittweisen Verschiebung der Gantry **2** ist die Gantry **2** über die Arretierungsvorrichtung **16** arretiert.

Zur computertomographischen Röntgenbildaufnahme der menschlichen Brust wird die Arretierungsvorrichtung **16** gelöst und die Gantry um 90° bezüglich der Darstellung in Fig. 1 geschwenkt und die Verschiebevorrichtung **17** arretiert. Dies entspricht einer Schwenkung der Mittelachse **z** um 90°. Mit der Hebevorrichtung **18** ist somit die Gantry **2** in Richtung der Mittelachse **z** verschiebbar. Eine computertomographische Röntgenbildaufnahme einer menschlichen Brust erfolgt beispielsweise dadurch, dass eine Patientin auf einer Liege mit einer Aussparung liegt, wobei eine Brust der Patientin in der Aussparung der Liege und zwischen den Röntgenemittern **3** und Röntgendetektoren **4** platziert ist.

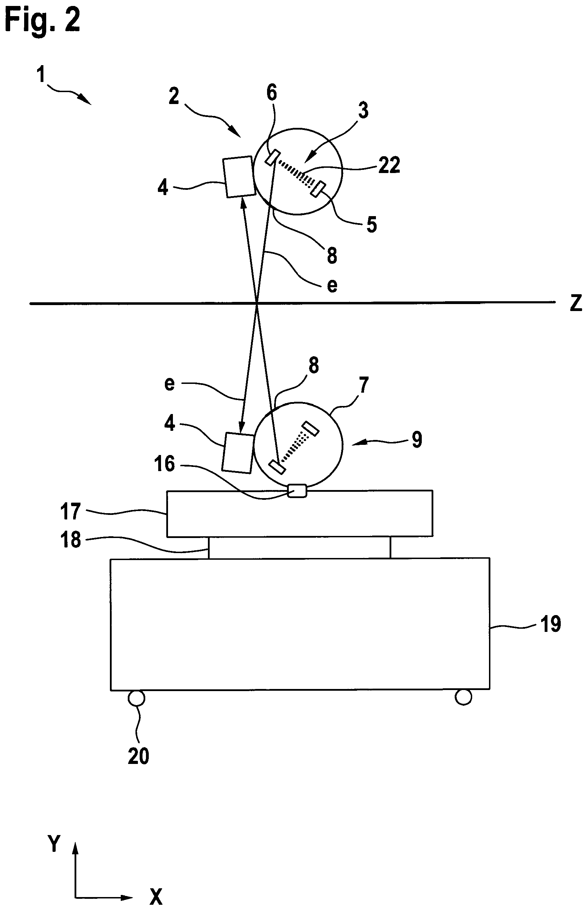

Die Fig. 2 zeigt dasselbe Ausführungsbeispiel wie in Fig. 1, wobei der Computertomograph **1** im Querschnitt parallel zur Mittelachse **z** seiner Gantry **2** dargestellt ist. In der Fig. 2 ist die Projektion des mit der Kathode **5** erzeugten Elektronen-Strahlenbündels **22** auf die Anode **6** und die Verläufe der Hauptemissionsrichtungen **e** der erzeugten Röntgenstrahlung exemplarisch dargestellt. Die Fig. 2 ist nicht maßstabsgetreu. In der Fig. 2 ist die Halterung **14** mit den zwei Schwenkpunkten **15** nicht sichtbar.

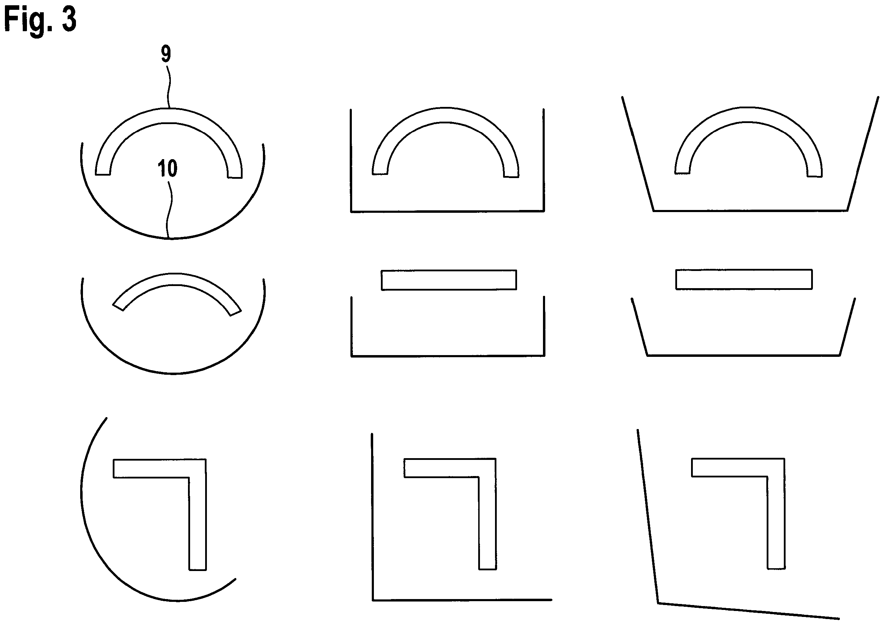

Die Fig. 3 zeigt schematisch einen Computertomographen **1** in verschiedenen Ausführungen bezüglich der Anordnungen von Röntgenemittern **3** und Röntgendetektoren **4** zueinander. Hierbei entsprechen die Geometrien der jeweiligen MBFEX-Röhren **9** und der Detektor-Baugruppe **10** den jeweiligen Anordnungsgeometrien der Röntgenemitter **3** und Röntgendetektoren **4.** Wie aus den einzelnen Darstellungen hervorgeht, kann die MBFEX-Röhre **9** eine kreisbogenförmig gekrümmte, gerade oder geknickte Form aufweisen. Die Detektor-Baugruppe **10** kann ebenfalls kreisbogenförmig gekrümmt sein. Ebenso kann die Detektor-Baugruppe **10,** welche in den piktogrammartigen Darstellungen in Fig. 3 jeweils lediglich in Form einer Linie skizziert ist, eine einfach oder mehrfach geknickte Form aufweisen. In nicht dargestellter, an sich bekannter Weise kann eine Detektor-Baugruppe **10** auch komplett flach sein.

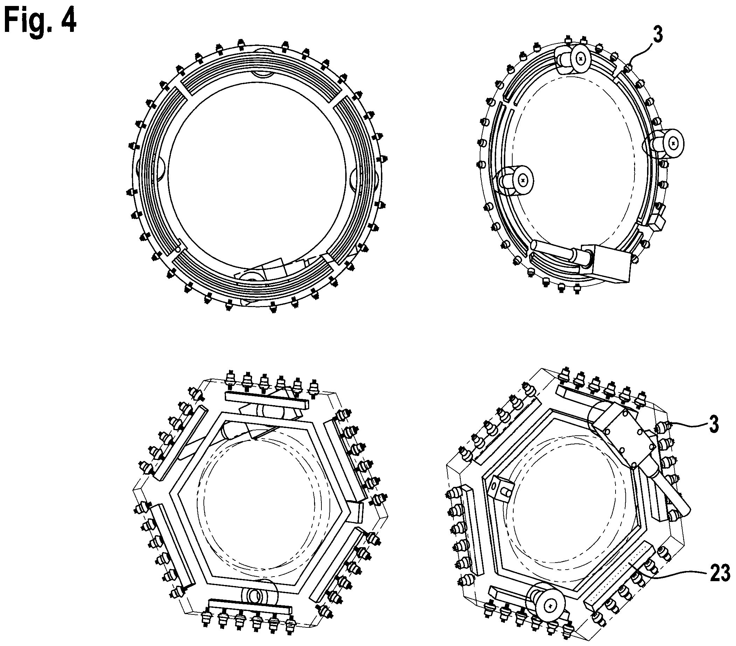

Die Fig. 4 zeigt zwei Ausführungsbeispiele des Computertomographen **1** jeweils mit Sicht auf die MBFEX-Röhre **9.** In dem ersten Ausführungsbeispiel (Fig. 4 links und rechts oben) sind die Röntgenemitter **3** in der MBFEX-Röhre **9** kreisförmig um die Mittelachse **z** angeordnet. In dem zweiten Ausführungsbeispiel (Fig. 4 links und rechts unten) sind die Röntgenemitter **3** in der MBFEX-Röhre **9** aus sechs gleichförmigen Zeilen **23** zu einem regelmäßigen Hexagon ausgebildet, wobei auf jeder Zeile **23** jeweils sechs Röntgenemitter **3** angeordnet sind. Die zu den jeweiligen Gantrys **2** zugehörigen Detektor-Baugruppen **10** der beiden Ausführungsbeispiele sind in der Fig. 4 nicht sichtbar; die Detektor-Baugruppen **10** weisen aber dieselbe Geometrie wie die jeweiligen MBFEX-Röhren **9** auf, wobei die Anordnungsgeometrie der Röntgendetektoren **4** hierbei derjenigen der Anordnungsgeometrie der Röntgenemitter **3** entspricht.

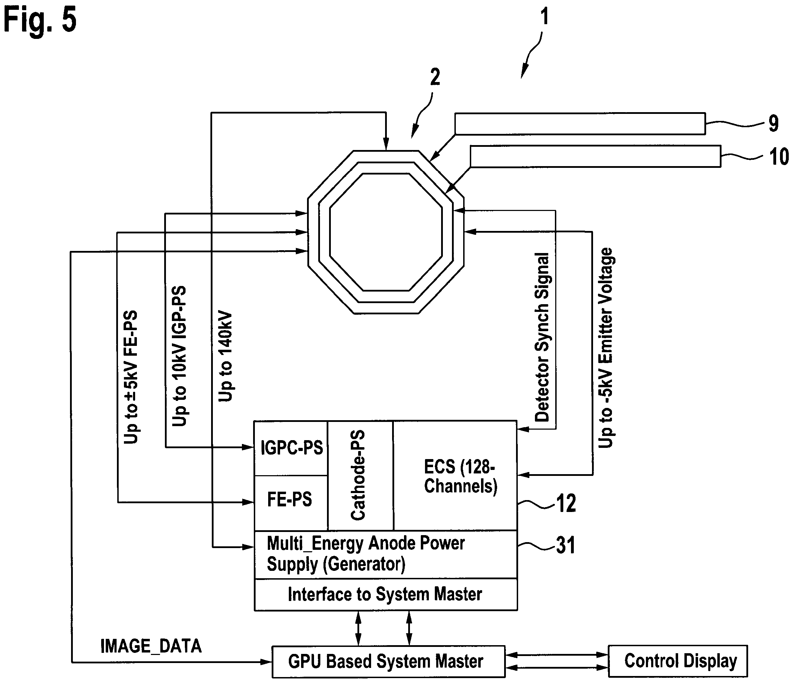

Die Fig. 5 zeigt ein Ausführungsbeispiel des Computertomographen **1** mit schematischer Darstellung eines elektronischen Mehrkanal-Steuersystems **12.** Das elektronische Mehrkanal-Steuersystem **12** ist zur Bedienung jedes Röntgenemitters im gepulsten Betriebsmodus vorgesehen, wobei das Mehrkanal-Steuersystem **12** eine Hochfrequenz-Hochspannungs-Anodenstromversorgung sowie eine schnelle Feedback-Schleife zwischen Röntgenstrahl und elektronischem Steuersystem für die Strommessung aufweist, um eine präzise und gleichbleibende Dosissteuerung (mAs) von Röntgenemitter **3** zu Röntgenemitter **3** zu ermöglichen. Der Rekonstruktionsalgorithmus basiert auf der gefilterten Rückprojektion mit der Option, die Vorteile des iterativen Rekonstruktionsalgorithmus dafür zu nutzen, die Anzahl der Ansichten pro Aufnahme zu senken und automatisch die Expositionsdosis für den Patienten pro Aufnahme zu verringern.

Im Ausführungsbeispiel nach Fig. 5 weist die Gantry **2** 128 Röntgenemitter **3** und eine Vielzahl von Röntgendetektoren **4** auf. Somit sind in diesem Ausführungsbeispiel 128 Projektionen erzeugbar. In der MBFEX-Röhre **9** ist aus acht gleichförmigen Zeilen **23** von Röntgenemittern **3** zu einem regelmäßigen Oktagon ausgebildet, wobei auf jeder Zeile **23** jeweils 16 Röntgenemitter **3** angeordnet sind. Die Röntgendetektoren **4** umfassende Detektor-Baugruppe **10** ist ebenfalls aus acht gleichförmigen Zeilen **23** zu einem regelmäßigen Oktagon ausgebildet, wobei ebenfalls auf jeder Zeile **23** jeweils eine gleiche Anzahl von Röntgendetektoren **4** angeordnet sind. Die Röntgenemitter **3** und die Röntgendetektoren **4** sind in der Fig. 5 nicht sichtbar.

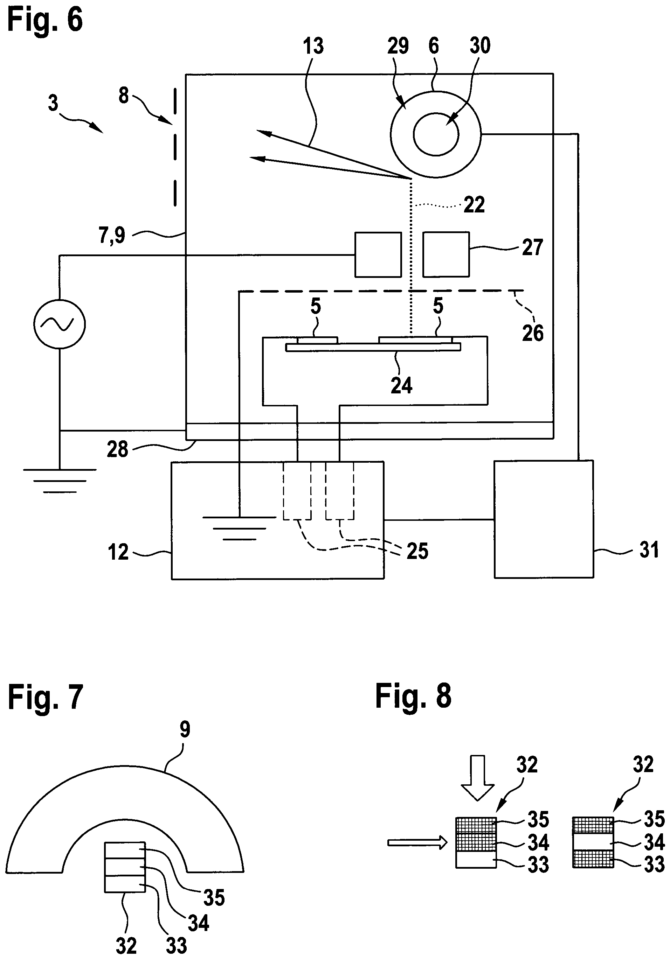

Einzelheiten der MBFEX-Röhre **9** des Computertomographen **1** nach Fig. 1 werden im Folgenden anhand Fig. 6 erläutert.

Innerhalb der Vakuumröhre **7** sind mehrere Kathoden **5,** das heißt Elektronenemitter, erkennbar, welche sich hinsichtlich ihrer Geometrie voneinander unterscheiden und auf einer gemeinsamen Platine **24** angeordnet sind. Jede Kathode **5** ist an eine separate Emitteransteuerung **25** angeschlossen. Die einzelnen Emitteransteuerungen **25** sind in das elektronische Mehrkanal-Steuersystem **12** integriert und erlauben eine individuelle Ansteuerung der Kathoden **5.** Die Kathoden **5** werden auf negativem Potential betrieben.

Ein von einer Kathode **5** ausgehendes Elektronen-Strahlenbündel **22** wird mit Hilfe eines Extraktionsgitters **26** emittiert, wobei ein gemeinsames Extraktionsgitter **26** mit einer Mehrzahl an Kathoden **5** zusammenwirkt. Das Extraktionsgitter **26** ist, wie aus Fig. 6 hervorgeht, über das elektronische Mehrkanal-Steuersystem **12** geerdet.

Im Unterschied hierzu ist eine Fokussierungselektrode **27,** welche allgemein als Strahlungsbeeinflussungsmittel bezeichnet wird, über das mit **28** bezeichnete Röhrengehäuse der MBFEX-Röhre **9** geerdet. Die separate Erdung des Extraktionsgitters **26** unabhängig von der Fokussierungselektrode **27** hat Vorteile hinsichtlich der Betriebsstabilität des Röntgenemitters **3.** Zwischen der mittels der Emitteransteuerungen **25** an die Kathoden **5** angelegten Spannung und dem Emissionsstrom existiert ein näherungsweise exponentieller Zusammenhang. Dies bedeutet, dass der in Form des Elektronen-Strahlenbündel **22** existierende elektrische Strom sehr empfindlich auf Erhöhungen der an den Kathoden **5** anliegenden Emitterspannung reagiert. Gerät der Emissionsstrom in einen zu hohen Bereich, so erzeugt das Elektronen-Strahlenbündel **22** ein vom Brennfleck auf der Anode **6** ausgehendes Ionen-Bombardement, welches auf das Extraktionsgitter **26** einwirkt. Trotz der Erdung der Fokussierungselektrode **27** wird das Potential der Fokussierungselektrode **27** dadurch kurzfristig in einen positiven Bereich gezogen. Durch die Entkopplung der Erdung des Extraktionsgitters **26** von der Erdung der Fokussierungselektrode **27** werden weitere Rückwirkungen auf das Extraktionsgitter **26,** welche eine schlagartige, starke Erhöhung des Emissionsstroms zur Folge hätten, wirksam unterdrückt.

Abweichend vom in Fig. 6 dargestellten Ausführungsbeispiel mit passiver Fokussierungselektrode **27** kann der Röntgenemitter **3** auch mit aktiver Fokussierungselektrode **27** betrieben werden. In jedem Fall ist die Fokussierung des Elektronen-Strahlenbündels **22** derart gestaltet, dass ein fokussierender Effekt in mehreren, zueinander orthogonalen Richtungen gegeben ist.

Die Anode **6,** auf welche das Elektronen-Strahlenbündel **22** auftrifft, ist als nicht rotierende, ölgekühlte Anode gestaltet. Hierbei strömt ein Kühlmittel, nämlich ein leitfähiges Öl, durch einen äußeren Kanal **29** in die Anode **6** ein und durch einen inneren Kanal **30** aus der Anode **6** aus. Die Kanäle **29, 30** sind konzentrisch in der Anode **6** angeordnet. Die Kanäle **29, 30** sind in nicht dargestellter Weise durch das Röhrengehäuse **28** der MBFEX-Röhre **9** hindurchgeführt. Sofern die Anode **6** insgesamt eine stabförmige, gerade Form aufweist, kann sie in abweichender Ausführungsform auch als rotierende Anode gestaltet sein, wobei die Mittelachse der Anode in diesem Fall zugleich die Rotationsachse darstellt.

Die Anode **6** ist an eine Anodenansteuerung **31** angeschlossen, welche sowohl die Energieversorgung der Anode **6** sicherstellt als auch den Wert des Anodenstroms bereitstellt. Dieser Wert wird an das gemäß Fig. 6 separat ausgeführte elektronische Mehrkanal-Steuersystem **12** weitergeleitet, so dass ein geschlossener Regelkreis gebildet ist, mit welchem eine strombasierte Stromregelung des Röntgenemitters **3** realisiert ist. Auch durch das Extraktionsgitter **26** sowie durch die Fokussierungselektrode **27** abfließende elektrische Ströme werden in dieser Regelung berücksichtigt.

In Fig. 7 ist die mögliche Lage eines Untersuchungsobjektes **32** relativ zur MBFEX-Röhre **9** skizziert. Das Untersuchungsobjekt **32** weist verschiedene Volumenbereiche **33, 34, 35** auf. Die Verteilung von Röntgenstrahlen absorbierendem Material innerhalb der Volumenbereiche **33**, **34, 35** sei variabel, wie in Fig. 8 skizziert ist. Mögliche Projektionsrichtungen, in welchen Projektionsbilder des Untersuchungsobjektes **32** aufgenommen werden können, sind in Fig. 8 durch Pfeile visualisiert.

In Fig. 8 ist links ein erster Zustand und rechts ein zweiter Zustand des Untersuchungsobjektes **32** skizziert. Wie aus den symbolisierten Darstellungen hervorgeht, befindet sich im links dargestellten Zustand dichtes Material, das heißt Röntgenstrahlung stark absorbierendes Material, in den Volumenbereichen **34** und **35.** Dagegen ist solches Material im zweiten Zustand des Untersuchungsobjektes **32** ausschließlich in den Volumenbereichen **33** und **35** verteilt. Im Übrigen sei das Untersuchungsobjekt **32** im Wesentlichen frei von Röntgenstrahlung absorbierendem Material.

Wird ein Projektionsbild des Untersuchungsobjektes **32,** bezogen auf die Anordnung nach den Figuren 7 und 8, mit vertikaler Projektionsrichtung generiert, so ist die Änderung vom ersten zum zweiten Zustand nicht erkennbar. Dagegen ist diese Änderung bei horizontaler Projektionsrichtung in vollem Umfang sichtbar. Diesem Umstand wird beim Betrieb des Computertomographen **1** dadurch Rechnung getragen, dass im Laufe der Ansteuerung verschiedener Kathoden **5** solche Kathoden **5** häufiger aktiviert werden, die Änderungen des Untersuchungsobjektes **32** besonders deutlich zu Tage treten lassen. Die Auswahl der Kathoden **5** erfolgt hierbei automatisch während der laufenden Aufnahme des Untersuchungsobjektes **32** auf Basis der permanent durchgeführten Bildauswertung.

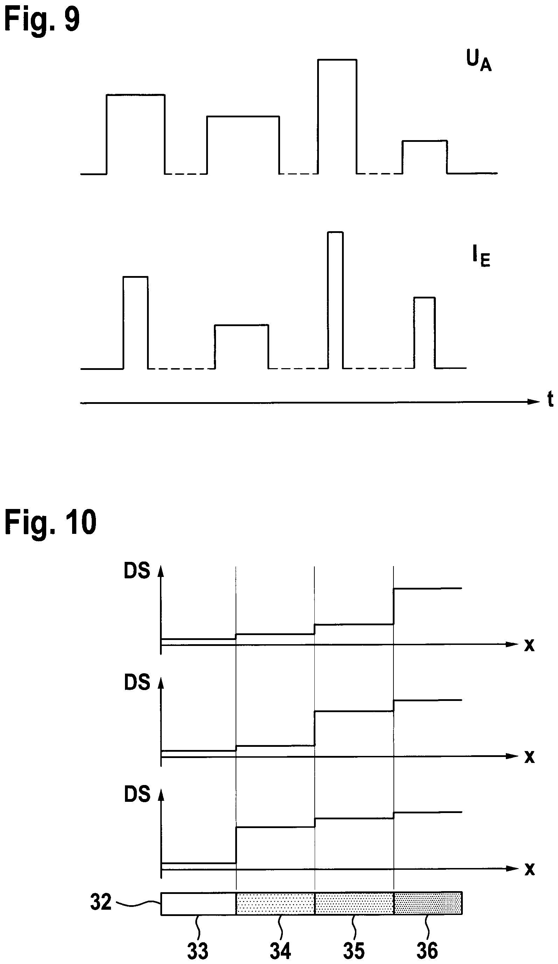

Die Fig. 9 veranschaulicht Möglichkeiten der Variierung sowohl der Anodenspannung U_{A}, welche an der Anode **6** anliegt, als auch des Emitterstroms I_{E}, welcher von einer Kathode **5** ausgeht. Die Zeitskala, welche in Fig. 9 den gepulsten Betrieb des Röntgenemitters **3** betrifft, ist in üblicher Weise mit t beschriftet und bezieht sich sowohl auf die Anodenspannung U_{A} als auch auf den Emitterstrom I_{E}. Bei insgesamt vier Pulsen beträgt im dargestellten Fall die Anodenspannung U_{A} 100 kV, 80 kV, 140 kV und 60 kV und der Emitterstrom I_{E}. 1 A, 0,5 A, 2 A und 0,8 A. Die sehr schnelle Veränderung der Anodenspannung U_{A} sowie des Emitterstroms I_{E} ermöglicht Multi-Energy-Aufnahmen eines Untersuchungsobjektes **32.**

Im Fall von Fig. 10 weist ein Untersuchungsobjekt **32** vier verschiedene Volumenbereiche **33, 34, 35, 36** mit zunehmender Dichte auf. Ein mit dem Röntgendetektor **3** erfasstes Detektorsignal ist mit DS bezeichnet. Es liefert eine Information über die Absorption der Röntgenstrahlung, welche üblicherweise in Hounsfield-Einheiten angegeben wird. Gemäß Fig. 10 werden Röntgenaufnahmen mit drei verschiedenen Energieeinstellungen der emittierten Röntgenstrahlung erstellt. In jedem der drei Fälle ist eine andere Kontur des Untersuchungsobjektes **32** besonders gut erkennbar, wie in den drei in Fig. 10 wiedergegebenen, auf eine gemeinsame Ortsachse bezogenen Diagrammen veranschaulicht ist. Insgesamt sind damit mit dem Computertomographen **1** Röntgenaufnahmen besonders hoher Qualität generierbar.

### Bezugszeichenliste

- 1: Computertomograph
- 2: Gantry
- 3: Röntgenemitter
- 4: Röntgendetektor
- 5: Kathode
- 6: Anode
- 7: Vakuumröhre
- 8: Röntgenfenster
- 9: MBFEX-Röhre
- 10: Detektor-Baugruppe
- 11: Gerätebasis
- 12: Elektronisches Mehrkanal-Steuersystem
- 13: Röntgenstrahlenfächer
- 14: Halterung
- 15: Schwenkpunkt
- 16: Arretierungsvorrichtung
- 17: Verschiebevorrichtung
- 18: Hebevorrichtung
- 19: Gehäuse
- 20: Rollen
- 21: Bildschirm
- 22: Elektronen-Strahlenbündel
- 23: Zeile
- 24: Platine
- 25: Emitteransteuerung
- 26: Extraktionsgitter
- 27: Fokussierungselektrode
- 28: Röhrengehäuse
- 29: äußerer Kanal
- 30: innerer Kanal
- 31: Anodenansteuerung
- 32: Untersuchungsobjekt
- 33: Volumenbereich
- 34: Volumenbereich
- 35: Volumenbereich
- 36: Volumenbereich

- DS: Detektorsignal
- e: Hauptemissionsrichtung
- I_{E}: Emitterstrom
- U_{A}: Anodenspannung
- z: Mittelachse

## Patentansprüche

1. Verfahren zum Betrieb eines Computertomographen (1), welcher eine rotationsfeste Gantry (2) aufweist, in der eine Mehrzahl von Röntgenemittern (3), Strahlungsbeeinflussungsmitteln (27) und Röntgendetektoren (4) starr angeordnet sind, wobei die Röntgenemitter (3) mehrere mit einer gemeinsamen Anode (6) zusammenwirkende Elektronenemitter (5) aufweisen und aus Projektionsbildern eines sich verändernden Untersuchungsobjektes (32) Schnittbilder generiert werden,
wobei ein erster Satz an Projektionsbildern, aufgenommen aus unterschiedlichen Projektionsrichtungen, generiert wird, und mindestens ein zusätzlicher Satz an Projektionsbildern aufgenommen wird, wobei die Projektionsrichtungen zumindest teilweise den Projektionsrichtungen des ersten Satzes an Projektionsbildern entsprechen,
**dadurch gekennzeichnet, dass** zwischen jeweils mindestens zwei aus übereinstimmender Projektionsrichtung aufgenommenen Projektionsbildern der Grad an Übereinstimmung festgestellt wird und weitere Projektionsbilder generiert werden, wobei die Häufigkeit der gewählten Projektionsrichtungen von dem Grad der Übereinstimmung der aus den betreffenden Projektionsrichtungen zu aufeinander folgenden Zeitpunkten aufgenommenen Projektionsbildern abhängt.

2. Verfahren nach Anspruch 1, **dadurch gekennzeichnet, dass** umso häufiger Projektionsaufnahmen aus einer bestimmten Projektionsrichtung erstellt werden, je geringer der Grad an Übereinstimmung zwischen zu aufeinander folgenden Zeitpunkten aus der entsprechenden Projektionsrichtung aufgenommenen Projektionsbildern ist.

3. Verfahren nach Anspruch 1 oder 2, **dadurch gekennzeichnet, dass** bei aufeinander folgenden Röntgenpulsen sowohl der Emissionsstrom (I_{E}) des Elektronenemitters (5) als auch die Anodenspannung (U_{A}) variiert wird.

4. Verfahren nach Anspruch 3, **dadurch gekennzeichnet, dass** die emittierte Röntgenstrahlung, was die Parameter Wellenlänge und Dosis pro Puls betrifft, in mindestens 100 Stufen während der Untersuchung eines Untersuchungsobjektes (32) variiert wird.

5. Verfahren nach einem der Ansprüche 1 bis 4, **dadurch gekennzeichnet, dass** die Röntgenemitter (3) Nanostäbchen enthaltende Kathoden (5) aufweisen, welche per Feldemission Elektronen emittieren, die auf eine starre, flüssigkeitsgekühlte Anode (6) treffen.

6. Verfahren nach Anspruch 5, **dadurch gekennzeichnet, dass** die Elektronen durch Kohlenstoffnanoröhren enthaltende Kathoden (5) emittiert werden.

7. Verfahren nach einem der Ansprüche 1 bis 6, **dadurch gekennzeichnet, dass** die Elektronen durch Röntgenemitter (3) emittiert werden, welche - ebenso wie Röntgendetektoren (4) - eine geometrische Mittelachse (z) des Computertomographen (1) vollständig umschließen.

8. Verfahren nach Anspruch 7, **dadurch gekennzeichnet, dass** die Elektronen durch Röntgenemitter (3) emittiert werden, welche - ebenso wie die Röntgendetektoren (4) - auf einem Kreis angeordnet sind.

9. Verfahren nach Anspruch 7, **dadurch gekennzeichnet, dass** die Elektronen durch Röntgenemitter (3) emittiert werden, welche - ebenso wie die Röntgendetektoren (4) - in mindestens drei gleich langen Zeilen (23) angeordnet sind, wobei die Zeilen (23) ein regelmäßiges Polygon ausbilden.

10. Verfahren nach einem der Ansprüche 1 bis 9, **dadurch gekennzeichnet, dass** Röntgenstrahlung mittels Röntgendetektoren (4), welche direkte Halbleiterdetektoren aufweisen, detektiert wird.

11. Verfahren nach einem der Ansprüche 1 bis 10, **dadurch gekennzeichnet, dass** Elektronen von mindestens acht, einem gemeinsamen Extraktionsgitter (26) zugeordneten Kathoden (5) emittiert werden.

12. Verfahren nach einem der Ansprüche 1 bis 11, **dadurch gekennzeichnet, dass** Elektronen von mindestens zwei unterschiedlichen Kathoden (5) emittiert werden.

## Claims

1. A method for operating a computer tomograph (1), which has a rotationally fixed gantry (2), in which a plurality of X-ray emitters (3), radiation influencing means (27), and X-ray detectors (4) are rigidly arranged, wherein the X-ray emitters (3) have several electron emitters (5) cooperating with a common anode (6), and sectional images are formed from projection images of a changing object to be examined (32),
wherein a first set of projection images, recorded from different projection directions, is generated, and at least one additional set of projection images is recorded,
wherein the projection directions at least partially correspond to the projection directions of the first set of projection images,
**characterized in that**
the degree of conformity between at least two respective projection images recorded from corresponding projection directions is determined and further projection images are generated,
wherein the frequency of the selected projection directions is a function of the level of the conformity of the projection images recorded from the respective projection directions at consecutive points in time.

2. The method according to claim 1, **characterized in that** the more frequently projection images are generated from a certain projection direction, the smaller the level of conformity between projection images recorded from the corresponding projection direction at consecutive points in time.

3. The method according to claim 1 or 2, **characterized in that** the emission current (I_{E}) of the electron emitter (5) as well as the anode voltage (U_{A}) is varied in response to consecutive X-ray pulses.

4. The method according to claim 3, **characterized in that** with respect to the parameters wavelength and dose per pulse, the emitted X-ray radiation is varied in at least 100 stages during the examination of an object to be examined (32).

5. The method according to any one of claims 1 to 4, **characterized in that** the X-ray emitters (3) have cathodes (5) containing nanorods, which, via field emission, emit electrons, which impinge on a rigid, liquid-cooled anode (6).

6. The method according to claim 5, **characterized in that** the electrons are emitted by means of cathodes (5) containing carbon nanotubes.

7. The method according to any one of claims 1 to 6, **characterized in that** the electrons are emitted by means of X-ray emitters (3), which, just like X-ray detectors (4), completely enclose a geometric central axis (z) of the computer tomograph (1).

8. The method according to claim 7, **characterized in that** the electrons are emitted by means of X-ray emitters (3), which, just like the X-ray detectors (4), are arranged on a circle.

9. The method according to claim 7, **characterized in that** the electrons are emitted by means of X-ray emitters (3), which, just like the X-ray detectors (4), are arranged in at least three rows (23) of equal lengths, wherein the rows (23) form a regular polygon.

10. The method according to any one of claims 1 to 9, **characterized in that** X-ray radiation is detected by means of X-ray detectors (4), which have direct semiconductor detectors.

11. The method according to any one of claims 1 to 10, **characterized in that** electrons of at least eight cathodes (5) assigned to a common extraction grid (26) are emitted.

12. The method according to any one of claims 1 to 11, **characterized in that** electrons of at least two different cathodes (5) are emitted.

## Revendications

1. Procédé d'exploitation d'un tomodensitomètre (1) qui présente un portique (2) fixe en rotation, dans lequel sont disposés rigidement une majorité d'émetteurs de rayons X (3), de moyens influençant le rayonnement (27) et de détecteurs de rayons X (4), les émetteurs de rayons X (3) présentant plusieurs émetteurs d'électrons (5) interagissant avec une anode (6) commune et des vues en coupe étant générées à partir d'images de projection d'un objet d'examen (32) changeant,
un premier ensemble d'images de projection, enregistré à partir de directions de projection différentes, étant généré et
au moins un ensemble supplémentaire d'images de projection étant enregistré,
les directions de projection correspondant au moins en partie aux directions de projection du premier ensemble d'images de projection,
**caractérisé en ce que**,
entre au moins deux images de projection enregistrées à partir de directions de projection correspondantes, le degré de correspondance est constaté et
d'autres images de projection sont générées,
la fréquence des directions de projection sélectionnées étant fonction du degré de correspondance des images de projection enregistrées à partir des directions de projection respectives à des moments successifs.

2. Procédé conformément à la revendication 1, **caractérisé en ce que** plus souvent des vues de projection sont établies à partir d'une direction de projection définie, plus le degré de correspondance entre des images de projection enregistrées à partir de la direction de projection correspondante à des moments successifs est faible.

3. Procédé conformément à la revendication 1 ou 2,
**caractérisé en ce que** le courant d'émission (I_{E}) de l'émetteur d'électrons (5) et la tension anodique (U_{A}) peuvent varier avec des impulsions de rayons X successives.

4. Procédé conformément à la revendication 3, **caractérisé en ce que** les rayons X émis quant aux paramètres Longueur d'onde et Dose par impulsion peuvent varier dans au moins 100 niveaux pendant l'examen d'un objet d'examen (32).

5. Procédé conformément à l'une des revendications 1 à 4,
**caractérisé en ce que** les émetteurs de rayons X (3) présentent des cathodes (5) contenant des nanotiges qui émettent des électrons par émission par effet de champ lesquels rencontrent une anode (6) rigide, refroidie par liquide.

6. Procédé conformément à la revendication 5, **caractérisé en ce que** les électrons sont émis par des cathodes (5) contenant des nanotubes de carbone.

7. Procédé conformément à l'une des revendications 1 à 6,
**caractérisé en ce que** les électrons sont émis par des émetteurs de rayons X (3) qui, comme les détecteurs de rayons X (4), entourent complètement un axe géométrique central (z) du tomodensitomètre (1).

8. Procédé conformément à la revendication 7, **caractérisé en ce que** les électrons sont émis par des émetteurs de rayons X (3) qui, comme les détecteurs de rayons X (4), sont disposés sur un cercle.

9. Procédé conformément à la revendication 7, **caractérisé en ce que** les électrons sont émis par des émetteurs de rayons X (3) qui, comme les détecteurs de rayons X (4), sont disposés dans au moins trois lignes (23) de même longueur, les lignes (23) formant un polygone régulier.

10. Procédé conformément à l'une des revendications 1 à 9,
**caractérisé en ce que** les rayons X sont détectés au moyen de détecteurs de rayons X (4) qui présentent des détecteurs à semiconducteurs directs.

11. Procédé conformément à l'une des revendications 1 à 10,
**caractérisé en ce que** des électrons sont émis par au moins huit cathodes (5) affectées à une grille d'extraction commune (26).

12. Procédé conformément à l'une des revendications 1 à 11,
**caractérisé en ce que** des électrons sont émis par au moins deux cathodes (5) différentes
